# EUROPEAN PATENT SPECIFICATION

(11) **EP 1 644 181 B1**
(45) Date of publication and mention of the grant of the patent: **13.11.2013**
(21) Application number: 03729719.9
(22) Date of filing: 25.06.2003
(51) Int. Cl.: A41F 1/00, A61F 2/50, A61F 2/66, B29C 70/20, B29C 70/34, B29C 70/54, B32B 5/28, B32B 27/12, B32B 27/38

(54) **Method of construction of moulded products**
Herstellungsverfahren für Formkörper
Procédé de production de produits moules

(43) Date of publication of application: 12.04.2006
(73) Proprietor: ProsMedix International Pte. Ltd., Singapore 415934 (SG)
(72) Inventor:
(74) Representative: Miller, James Lionel Woolverton
(86) International application number: PCT/AU2003/000796
(87) International publication number: WO 2004/113058

(56) References cited:
- EP-A2- 0 432 141
- WO-A1-01/76892
- WO-A1-96/08216
- CA-A1- 2 035 551
- DE-A1- 3 808 994
- GB-A- 1 364 076
- JP-A- 2 153 938
- US-A- 4 031 181
- US-A- 4 167 429
- US-A- 4 376 749
- US-A- 4 397 048
- US-A- 4 413 860
- US-A- 4 547 913
- US-A- 4 568 582
- US-A- 4 959 073
- US-A- 5 192 330
- US-A- 5 746 773
- US-A- 5 976 191
- US-A- 5 993 487
- US-A- 6 071 313
- US-B2- 6 562 075
- DATABASE WPI Derwent Publications Ltd., London, GB; Class A32, AN 2002-282553, XP002983255 & EP 1 074 369 A (MICHELIN RECH & TECH) 07 February 2001
- DATABASE WPI Derwent Publications Ltd., London, GB; Class A97, AN 1985-078927, XP002983256 & JP 60 032 615 A (MITSUBISHI RAYON KK) 19 February 1985
- DATABASE WPI Derwent Publications Ltd., London, GB; Class P73, AN 1988-143594, XP002023670 & JP 63 084 932 A (CIBA GEIGY AG) 05 May 1998
- DATABASE WPI Derwent Publications Ltd., London, GB; Class A32, AN 1992-214140, XP002983257 & JP 04 144 712 A (KUSUO S) 19 May 1992

## Description

### FIELD OF THE INVENTION

THIS INVENTION relates to a method of construction of moulded products. In particular, the invention relates to a method of construction of prosthetic devices and the specification will now describe in detail by way of example a method of construction of prosthetic devices although it should not be limited to this particular example.

### BACKGROUND ART

In the past 15 years, there have been an increasing number of lower-limb amputees who have desired to enhance their quality of life by being able to have access to an affordable prosthesis that will provide not only basic mobility but which will also encourage and permit participation in low- to high-impact activities in the same manner as enjoyed by non-amputees.

Numerous technological advances in the field of prosthetics have allowed this to happen and have provided a wide range of prosthetic legs to choose from. In coalition with the general type of prosthesis for normal gait activities, such as basic ambulant walking and work type activities, there has been a demand by amputees for prostheses that can enable them to lead a more active lifestyle and, thus, improve their quality of life. There has been much effort within the prosthetic industry to develop a lightweight and durable prosthesis that will allow amputees to participate in strenuous career options, such as laboring, farm work, etc, and various medium- to high-level activities, such as jogging, squash, climbing, tennis, etc, and very high-impact sports, such as running, sprinting, jumping, basketball and volleyball.

Numerous different prostheses have been developed, each trying to replicate the function and appearance of the replaced body part. Lower leg prostheses present unique problems. They must be strong enough to support a person's weight and activity level and to withstand the forces encountered through gait movement. As a result, some prostheses developed in the past were very heavy and rigid and restricted the wearer's activity level, career, recreational and sport options.

There are a number of different composite foot and leg prosthetic devices that have now been proposed, most of which utilize modern composite material technology to impart energy storage and release during use. Examples of prosthetic foot devices that have been fabricated with composite materials, with the intention of storing energy when the foot is placed on the ground and to release it and provide a lift or thrust as the foot is removed from the ground to aid in the patient's gait are disclosed in Abrogast et al. US Patent No 4,865,612, concerning an artificial foot having a pair of thin forwardly extending spring members connected to a keel and Shorter et al. US Patent No 5,116,383. US Patent No 5,116,383 describes an energy storing foot having a single piece carbon fibre reinforced keel having a lower spring portion connected at its posterior end as a cantilever to an upper ankle mounting portion. The energy storing foot is combined with a resilient ankle joint of the ball and socket type.

Other lower leg prosthetic devices in the prior art have employed springs, including multiple leaf springs or flat spring-like members, in an attempt to approximate the response and performance of a natural foot. Examples include Merlette US Patent No 4,959,073 and Phillips US Patent No 4,547,913, 4,822,363 and 5,776,205.

An example of the abovementioned lower leg prosthetic device is a prosthesis known as the Flex-Foot, which provides a composite strut and foot configuration, which is specifically described in US Patent No 4,547,913. The spring-like action imparted by the materials used in such devices results in additional mobility and comfort to the wearer. Prior art prosthetic feet that utilize a plurality of springs, such as in Phillips US Patent No 5,776,205, tend to rock under vertical load as the load is distributed separately to the springs.

Furthermore, the dynamic feet of the Flex-Foot system that are commercially available, do not have rigid ankle joints. However, this system embodied a rearward separate heel segment or heel portion secured to the main body of the foot, at the mid foot. While this system provides better performance than the former prostheses, this system embraces a plurality of components and joints which are less than optimum for transference of energy from heel strike through to toe-off because of the rearward heel segment deficiency of transmission of continual energy from a separate heel plate through to a separate toe portion on the J-pylon as described in US Patent No 4,822,363 and/or a full sole member described in US Patent No 5,976,191. This is because of the necessity to incorporate additional support members or increase in pylon thickness, which is necessary to provide suitable wearer support and prosthesis performance.

Prior art prosthetic feet with a separate heel spring member and/or foam/rubber resident material placed between foot and sole member tend to absorb vertical load either in the spring member or in the foam/rubber resilient material and, thus, dissipates the transfer of constant energy from heel strike to toe-off.

US Patent Nos 4,822,363 and 4,547,913 refer specifically to a J-shaped pylon made from the composite material connected to a flat spring-like rearward heel plate. US Patent No 4,959,073 refers to a J-shaped pylon made from the composite material that is integrally formed with a heel member. Reference also may be made to US Patent No 5,037,444, which refers to a prosthetic foot made from the composite material alternatively known as the so-called "Seattle Foot". These devices make great advances in gait movement and activity levels and have a higher strength and stiffness to weight ratio of graphite to other materials, which results in almost complete return of input or stored energy, lighter weight, higher fatigue strength, and minimal creep. However, these devices also possess some disadvantages. For example, because of the higher strength and stiffness to weight ratio of graphite as compared to other materials, local stiffness, jolting and braking occurs because of material lay-up fabrication methods, which interferes with smooth and even flexing of the components during the wearer's stride. Furthermore, while substantially lighter in weight, they have an inherent rigidity because of the higher tensile strength resulting in greater stiffness to weight ratio of graphite to other material and also have areas more prone to fatigue stress failure.

Prior art prosthetic feet that utilize a spring-loaded heel which operates on a spring separate from a spring in the toe section, such as in the Phillips and Merlette patents discussed above, effectively store energy in the heel, but are ineffective in transferring the energy from the heel to the toe portion of the prosthetic foot as the foot rolls forward during the gait cycle. These devices still require separate loading of a spring in the toe section. As a result, wearers notice a distinct and unnatural lag or hesitation in rolling the foot forward during the gait cycle, giving the foot an unnatural feel and causing an uneven stride.

Some prior art prosthetic feet, such as the devices of Phillips US Patent Nos 4,547,913 and 4,822,363, can accommodate torsion movement about the longitudinal axis of the shin portion, but the shape of the shin portion is designed for spring strength and breaking strength, not torsion movement.

The prior art devices of US Patent Nos 4,547,913, 4,822,363 and 4,959,073 and others are made up exclusively from a composite material comprising high strength graphite filaments in a high-toughness epoxy thermosetting resin, constructed from uni-directional (UD) longitudinal fibres in their main core with woven latitudinal or woven angular fibres on their outer layers to improve their torsion strength. The latitudinal or angular woven materials come in the form of woven sleeves. These woven sleeves can easily slide over and cover the UD longitudinal fibres that are in the main core. These outer layers are woven sleeves and all their fibre filaments are continuous from start to finish. If this was not the case, it would not be possible to weave these filaments into a woven sleeve. Usually, the main core and the woven sleeves were formed by a number of plies or laminae that were placed in a rigid mould shaped to the final configuration and, subsequently, cured.

This provides a result wherein each of the continuous filaments extend uninterrupted through an upper extremity, shin portion and foot portion of the prosthesis or from the top of leg portion downwardly through the ankle section, across the top of the foot or toe portion. Also, the heel plate and/or sole member have continuous filaments that extend uninterrupted from front extremity to heel extremity ie. all fibre filaments are continuous and uninterrupted longitudinally, latitudinal or angularly in their constructions. Similar construction methods are described in US Patent Nos 5,037,44, 5,156,631, 5,181,933, 5,493,456, 5,514,185 and 6,071,313.

Such construction methods have the following disadvantages, ie:
(a) It was necessary to thicken the upper extremity of the pylon to provide a rigid upper extremity. This may also be accomplished by expanding the width of said upper extremity;
(b) It was important to increase the number of plies or laminae and, thus, the thickness of the pylon to accommodate users as their weight or activity level increases;
(c) Additional laminates were added to maintain strength as the width of the pylon is reduced;
(d) It was considered important to achieve a gradual reduction in the thickness of the pylon in the various dynamic areas thereof such that the foot portion is reduced in thickness compared to the ankle portion, and the ankle portion is reduced in thickness compared to the shin portion.

The number of plies or laminations or layers varies in the different sections. As a consequence of (a), (b), (c) and (d) above, it was a general expectation from conventional methods of construction of prostheses as described above that, depending on the foot size and strength needed in a particular prosthesis, the number of plies employed can be increased or decreased as required. For example, fewer laminations may be required for a smaller prosthesis, or a prosthesis for a lightweight individual. A larger prosthesis, or a prosthesis for a heavier individual, may require more laminations. It was considered generally desirable to employ as few laminations as are needed for strength and stiffness to keep the prosthesis as light weight as practicable.

From the discussion of prior art methods of construction above, it was necessary in the conventional manufacturing process to provide a plurality of different moulds or provide changes in mould cavity dimensions when additional plies or laminae were used to increase thicknesses or widths of prostheses. In other words it was necessary to use different mould cavity dimensions to correspond to different weights or different weight categories of prosthesis. The necessity of different thicknesses or widths was necessary to provide a variety of prostheses with different dimensions, thereby providing a disparity of thicknesses or widths to accommodate variations of pliability rates to support different exoskeleton and endoskeleton frames, body types and activity requirements of amputees.

EP 0432141 describes a femoral stem for permanent implantation into the natural femur and a method to produce it.

### OBJECT OF THE INVENTION

It has now been found that the above deficiencies of conventional manufacturing processes of conventional moulded products such as prostheses may now be reduced or overcome by the advent of the present invention where variations in requirements of individual users inclusive of amputees can be obtained by a process, as described hereinafter, which has the capability to make moulded products inclusive of prostheses having a range of different weights or different weight categories in a single dimensional cavity mould, thus, making the process not only more efficient, but also less expensive.

### SUMMARY OF THE INVENTION

According to the present invention there is provided a method of construction of a moulded product which includes the steps of:
(a) impregnating non-metallic fibres in epoxy resin whereby said impregnated fibres may be arranged to form a composite sheet of said fibres; and
(b) cutting a plurality of pre-pregs having fibres arranged in a longitudinal, i.e. 0 degrees, or latitudinal orientation, i.e. 90 degrees, from an intermediate sheet obtained by cutting said composite sheet formed in step (a) at an angle of 0 degrees; or
(b') cutting the composite sheet formed in step (a) at a variety of different angles selected from the group consisting of 15 degrees, 22 ½ degrees, 30 degrees, 45 degrees and 60 degrees so to obtain an intermediate sheet having sloping sides with said angle to the vertical; folding the intermediate sheet upon itself to form a final folded sheet; cutting a plurality of pre-pregs having fibres arranged in intersecting orientation from said final folded sheet; and
(c) forming successive layers of pre-pregs alternating pre-pregs obtained from step (b) and pre-pregs obtained from step (b') so that said layers are arranged in stacked relationship in a mould cavity of a compression mould; and
(d) compression moulding at elevated temperature; and
(e) removing the moulded product from the compression mould;
wherein in step (c), pre-pregs with various combinations of carbon/carbon or carbon/glass fibres are used with variations in fibre area weight and fibre orientation are used so that step (d) is carried out in a mould cavity having the same dimensions for different weights or different weight categories of moulded product.

Preferably, in step (a) use is made of a drum winding technique whereby said impregnated fibres are wound onto a drum so as to provide the composite sheet formed of the fibres supported on a sheet of release paper on the drum, whereby upon removal of the release paper, the intermediate sheet used in steps (b) or (b') is obtained by cutting the composite sheet as it is supported on the drum.

Preferably, the cutting of the composite sheet is carried out by provision of cutting lines or grooves which are formed in an outer surface of the drum at an angle selected from the group consisting of 15 degrees, 22 ½ degrees, 30 degrees, 45 degrees and 60 degrees for obtaining the pre-pregs of intersecting fibres in step (b') and 0 degrees for obtaining the pre-pregs of longitudinal and latitudinal fibres in step (b).

Preferably, use is made of a hot melt technique wherein after hot melt resin impregnation of the fibres they are laid onto a continuous sheet of release paper to obtain the composite sheet and subsequently stored as rolls.

Preferably, in step (a) the composite sheet has all of its fibres arranged in the same or similar orientation or direction.

Preferably, in step (b) each pre-preg has all of its fibres arranged in a longitudinal orientation.

Preferably, in step (b') each pre-preg has all of its fibres arranged in an intersecting orientation.

Preferably, in step (b') the angle at which the composite sheet is cut is selected from 30 degrees and 45 degrees.

Preferably, in step (a) the composite sheet is formed by longitudinally and/or latitudinally oriented fibres so as to provide a sheet which is in the shape of a rectangle or square.

Another significant difference between the solution drum winding as discussed above and the hot melt technique as discussed above is that instead of the cutting lines or grooves being formed on the drum in the solution drum winding technique in contrast in the hot melt technique the desired angles (say 15°, 22^{1/2}°, 30°, 45° or 60 degrees) will have to be cut physically on the pre-preg sheets.

The method of construction of the invention allows the fabrication of a lightweight, inexpensive moulded product such as a prosthetic device made of composite material for amputees and, more particularly, to an improved lower limb prosthesis. Such a moulding method is effective for overcoming the limitations in the strength, dynamic endurance, weight problems of fibroglass and the stiffness/rigidity and fatigue limitations of prior art methods described above.

The method of construction of the invention using non-metallic fibres in combination with an epoxy resin ensures that all individual fibres are impregnated to allow better dynamic endurance and a reduction in weight and thickness whilst retaining the natural flexibility and response of its fibres to allow the prosthesis to have a smoother transition from heel strike through to toe-off. Another advantage is that it allows better shock dispersion of the resilient constructed matrix that dissipates loads during heel strike.

While the device is preferably constructed of epoxy resin reinforced with laminates of carbon and/or glass fibres, it may be constructed of other non-metallic fibres such as aramid fibre, or recently developed synthetic fibres.

The application also discloses a moulded product present only to assist in the understanding of the invention, wherein said moulded product is formed from composite materials having layers of non-metallic fibres impregnated with an epoxy thermosetting resin, wherein said moulded product is formed at least partly from layers of said fibres wherein at least some of the fibres are arranged in an intersecting orientation.

Preferably the moulded product is a pylon which suitably is J shaped.

Preferably, said pylon, at least in outer extremities thereof having regard to a lateral dimension or thickness of the pylon, is formed from a laminate of alternating layer(s) of said intersecting fibres with some layer(s) of said fibres arranged in longitudinal and latitudinal orientation.

The moulded product may also comprise a prosthetic device comprising the J-shaped pylon in combination with a sole plate wherein the sole plate incorporates an anterior toe section and posterior heel section. The sole plate may also be formed from the same composite material as the J-shaped pylon. The moulded product may also comprise the sole plate per se.

The J-shaped pylon may have an upper shin mounting portion, a lower shin portion and an ankle zone. The ankle zone may taper in thickness from an upper part adjacent to the lower shin portion towards a lower end of the ankle zone.

The upper shin mounting portion, preferably has a substantially constant thickness and width. The lower shin portion may have a width or lateral dimension that diverges outwardly as it approaches the ankle zone. The ankle zone, at or approaching a lower or free end thereof; may have a slight concave curvature.

Preferably, the sole plate is of substantial width compared to the ankle zone. It may comprise a heel portion and a toe portion. Preferably, the heel portion has a complementary or corresponding curvature to the ankle zone where they abut with each other.

A resilient sole pad may be affixed to an underside of the sole plate in any suitable manner, eg by adhesive or the use of fasteners. Preferably, the sole plate is capable of being mounted to the underside of the ankle zone at a designated point of 90 to 110mm from a toe end of the sole plate. Through the provision of two sole plates, it allows for the plurality of foot sizing ranging from 23cm-30cm. Suitably, for this purpose, the sole plate and ankle zone may be provided with holes which may be aligned to receive fasteners, such as bolts, there through to secure the sole plate to the ankle zone at selected positions. Suitably, two co-aligned holes are provided in the ankle zone and sole plate.

Preferably, the sole plate is of substantially the same width as the ankle zone. Preferably, the heel portion is also substantially curved suitably on substantially the same radius as the leading end of the toe portion.

The upper mounting shin portion of the J-shaped pylon may be secured to a mounting bracket, for example, by any suitable form of releasable attachment means, such as bolts or other suitable fasteners, to enable the upper mounting shin portion to be fixed to a distal end of a leg stump socket. The leg stump socket has a T-shaped bracket attachable thereto. Preferably, the bracket has a top flange, which is fixed to or fixable to the socket and a leg flange to which the mounting portion may be secured. Alternatively, the upper shin portion can be laminated directly to the distal end of the leg stump socket.

The prosthetic device stores energy upon heel strike of the sole plate, with weight applied in the gait cycle, thereby transferring energy during heel strike-forward to mid foot (flat foot) and releases energy at toe-off point of sole plate to provide a stable and controlled propelling lift or thrust to the prosthetic device to aid in achieving a natural gait

The sole plate being fixed to the ankle zone, as described above, is capable of transferring smooth constant energy release from the heel strike through to toe-off of the sole plate during the gait cycle without lag, hesitation, jolting and/or braking, as a result of its one-piece design and fabrication. This is in contrast to prior art devices, such as US Patent No 4,822,363, that incorporate a releasable and attachable sole plate which has to transfer energy release through two separate members (ie. sole plate and pylon) to achieve energy release during the gait cycle.

The prosthetic device will accommodate angled or uneven terrain without the use of a separate ankle joint through the characteristics of the device that facilitate a continuous smoother transference of stored energy from heel strike through to toe-off. The device may include a plurality of toe and heel sections, wherein each of the toe and heel sections may be buffed to facilitate foot sizing.

The prosthetic device is further capable of minimizing torsional movements and will accommodate stable and controlled lateral medial movements. Increased knee control allows increased ease of roll over of sole plate dissipating inertia and, thus, providing a smoother transition of movement regardless of the intensity of heel strike and mid-strike and toe-off.

Preferably, the J-shaped pylon has a shank comprising both the upper shin mounting portion and the lower shin portion that has a uniform thickness and width for all weight categories. It can be cut off where required and adjusted to the wearer without affecting the above stated performance factors.

### BRIEF DESCRIPTION OF THE DRAWINGS

In order that the invention may be more readily understood and put into practical effect, reference will now be made to the accompanying drawings that illustrate a moulded product in the form of a prosthetic device wherein:
FIG. 1 is a perspective view from the rear of the prosthetic device present only to assist in the understanding of the invention;
FIG. 2 is a side elevation of the prosthetic device fitted to a stump socket;
FIG. 3 is a front view of the prosthetic device detached from the stump socket and with mounting bracket removed;
FIG. 4 is a plan view along line A-A of FIG. 2;
FIG. 5 is a plan view of the sole plate;
FIGS. 6 and 7 are perspective views illustrating steps (a) and (b) of the method of the invention;
FIGS. 8 to 10 show drums that may be used in the method of the invention in FIGS. 6 and 7 having cutting lines or grooves arranged at varying orientations;
FIGS. 11 to 13 show the formation of pre-pregs with fibres arranged in longitudinal orientation;
FIGS. 14 to 18 show the formation of pre-pregs having fibres arranged in an intersecting orientation; and
FIG. 19 is a general flow chart of a preferred embodiment of the method of the invention.

### DETAILED DESCRIPTION OF THE REFERRED EMBODIMENT

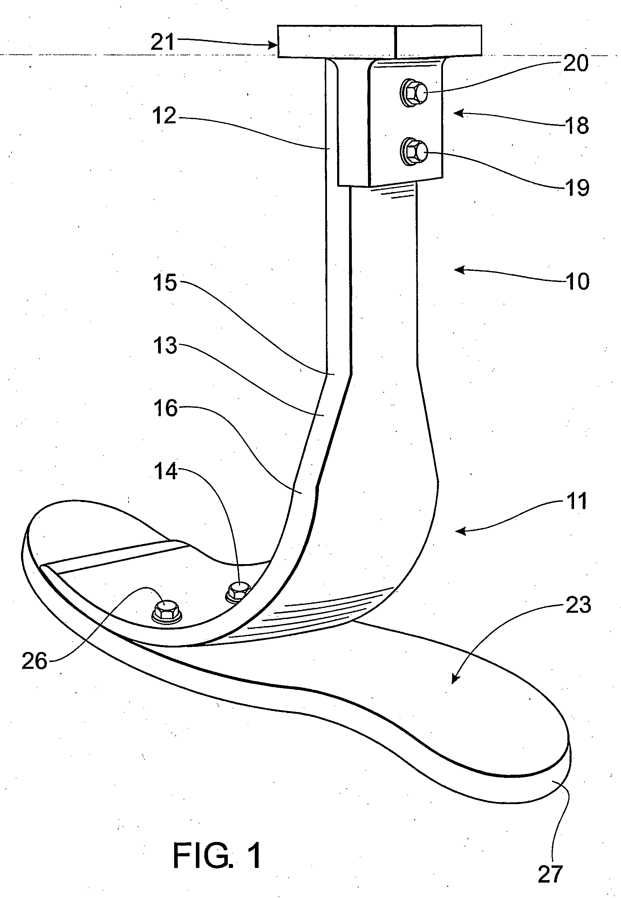

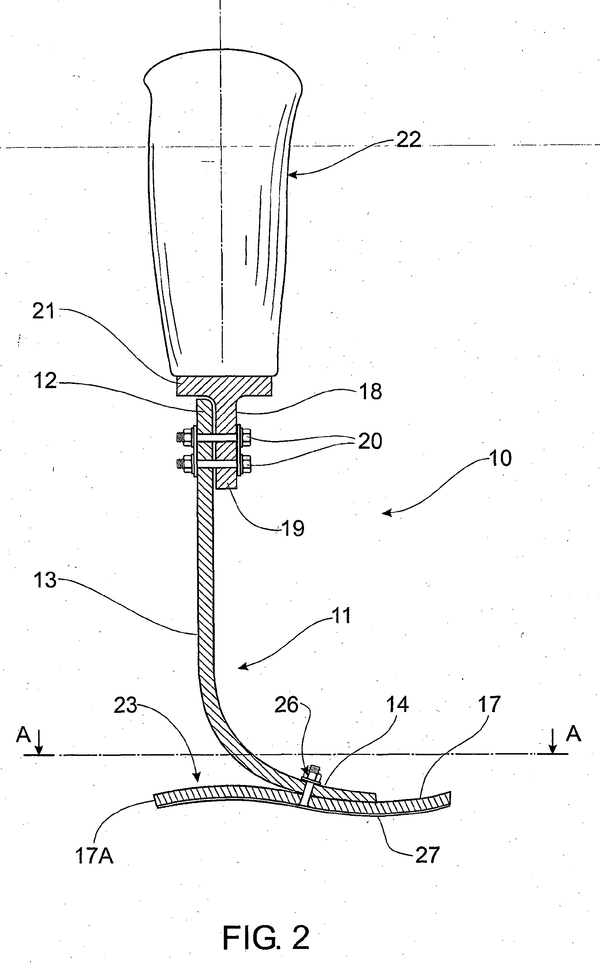

Referring to the drawings and, firstly, to FIGS. 1 and 2, there is illustrated a prosthetic lower limb device 10 having a pylon 11 of generally J-shaped configuration in side elevation and which has an upper shin mounting portion 12, a lower shin portion 13, and a ankle zone 14. As illustrated and as more clearly shown in FIG. 2, the upper shin mounting portion 12, the lower shin portion 13 and the ankle zone 14 are of substantially uniform thickness. There is also provided a sole plate.

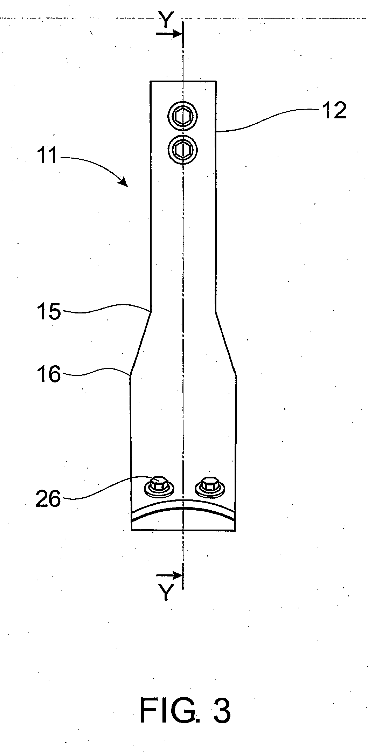

As best shown in FIG. 3, the J-shaped pylon 11 has a constant width from the upper mounting portion 12 to a point 15. The lower shin portion 13 diverges symmetrically outwards the longitudinal axis of pylon 11 designated by Y-Y, thereby increasing in width to a second point 16, from which the J-shaped pylon 11 continues at a substantially constant width forming ankle zone 14.

As shown in figs 1 to 2, the prosthetic lower limb device 10 also includes a T-shaped bracket 18 that has a leg 19 to which the upper mounting portion 12 of the J-shaped pylon 11 can be bolted via bolts 20. The T-shaped bracket 18 also includes a top flange 21 which can be laminated directly to a stump socket 22. Alternatively, the top flange 21 may have a variety of alignment connectors adjoined to it and then connected to the bottom of the stump socket 22 to provide adjustment and angle alignments to fine tune gait movements and complement socket fit.

Fixed to the underside of the ankle zone 14 is an integrally formed sole plate 23, which is a one-piece component that incorporates in tandem both the heel portion 17A and toe portion 17. The toe portion 17 is slightly concave on its upper side and extends forward from bolts 26 which interconnect pylon 11 to sole plate 23 while the heel portion 17A is slightly convex on its upper side and extends rearward from bolts 26. The sole plate 23 is substantially the same width as ankle zone 14. There is also provided a sole pad 27.

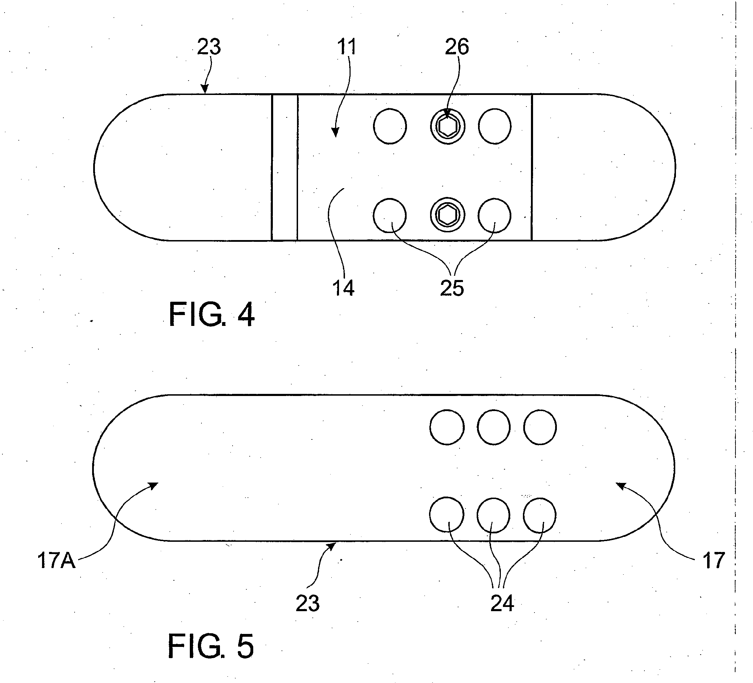

As shown also in FIGS. 4 and 5, there is provided in both sole plate 23 and pylon 11 a series of spaced pairs of co-aligned apertures 24 and 25 to permit the sole plate 23 to be secured to the ankle zone 14 by suitable bolts 26 through aligned apertures at a number of locations. As is apparent in FIG. 5, the leading end of the toe section 17 is of part circular form as is the heel end 17A of the sole plate 23. The above arrangements allow a variety of adjustments for the toe section 17 relative to the heel plate 23 of the prosthetic device 10, thus, permitting the toe section 17 and heel section 17A and sole plate 23 to accommodate subtle variations in the gait.

A resilient sole material, such as a rubber or urethane pad 27, may be affixed to the underside of the sole plate 23 covering both the toe portion 17 and the heel portion 17A, and it extends continuously along the entire length of the sole plate 23. The rubber/urethane pad 27 imparts flexibility to the prosthetic lower limb device 10 and helps to absorb heel strike or torque loads that may be applied at the heel or axially along the lower shin portion 13.

Further, the rubber or urethane pad 27 also provides energy dampening and increased comfort to the wearer. Typically, the rubber or urethane pad 27 is affixed in position by suitable adhesives. The rubber or urethane pad 27 can act as a non-slip surface to enable the prosthetic lower limb device 10 to be used without shoes.

The configuration and design of the main support or J-shaped pylon 11, as described above, enable all stress concentrations to be dispersed more evenly from the shin-mounting portion 12, through the lower shin portion 13 and into the ankle zone 14. The sole plate 23 is designed to provide greater stability and control of medial and lateral and for and aft movements.

Furthermore, the prosthesis of the present invention and method of construction also increases the intrinsic strength and durability of the prosthetic lower limb or leg, which facilitates uniformity in a single dimensional design for all weight categories as well as novelty of resiliency rejoinder and return energy of the total prosthesis.

It also facilitates distinctive variations of resiliency rates, smoother transition from heel impact to mid-strike to toe-off and allows better torsion movements, which can accommodate the many different exoskeleton and endoskeleton frames, body types and activity requirements of amputees, and a reduction in thigh and lower-back muscle fatigue and strain, compared to other prior art prostheses.

The resulting J-shaped pylon 11 preferably has uniformity of thickness that extends from the upper mounting portion 12 and extends through the lower shin portion 13 through the ankle zone 14. The shape of sole plate 23 as shown facilitates smoother controlled toe-off. Furthermore, the configuration and construction of the J-shaped pylon 11 provides effective resilience and return energy of the total prosthesis 10 from upper shin mounting portion 12 through to the ankle zone 14 with the improved process of manufacture avoiding the necessity of prior art prosthesis require a variety of distinctive dimensional (width and/or thickness) pylons, heel plates/sole members to vary their rigidity.

Typically, the length of the J-shaped pylon 11 to the lower surface of ankle zone 14 can vary from 255mm to 368mm. This, again, can be heightened through an increase of sole thickness (maximum 20mm), attachment of extension wedges to the top of T- shaped bracket 18 (3cm) and/or extension to the underside of the socket 22 (maximum 7.5cm). These methods of increasing prosthesis lower limb height will not influence performance of the prosthetic lower limb 10. The width of the J-shaped pylon 11 typically increases from 45mm at the mounting portion 12 and diverges outwardly at the point 15 to the position 16 (for a length of 80mm) to a width of 67mm, which then stays constant until ankle zone 14. Typically, the sole plate 23 also has a width of 67mm.

The centres for holes 24 in FIG. 5 and holes 25 in FIG. 4 are typically positioned 15mm from the side edges of the sole plate 23 and ankle zone 14, respectively. The holes 24 in the sole plate 23 may be positioned between 90mm and 110mm from the front edge of the plate 23 while the holes 25 in the ankle zone 14 are suitably co-aligned with the holes 24 in the sole plate 23. This will allow for an increase or decrease in foot sizing. It should be highlighted that the above dimensions may be varied.

The length of the upper shin-mounting portion 12 can be cut no less than 55mm to point 15 of the lower shin portion as shown in FIG 3 and adjusted to the wearer's requirement without affecting its performance. All of the J-shaped pylon 11 and sole plate 23 substantially have the same thickness, width and other dimensions throughout all weight classes from 30kg to 130kg at 10kg intervals. This is achieved through the novel method of the invention.

In the present invention, single-dimensioned mould cavity moulds may be used for the moulding of J-shaped pylon 11 and sole plate 23. Thus, the J-shaped pylon will have uniform dimensions in thickness, in width, shape and sizes for all classes of weight categories. Similarly, the sole plate 23 will also have uniformity in dimensions for thickness, width, shape and sizes for all classes of weight categories.

The toe section 17 and heel section 17A of the sole plate 23 can be buffed down to the appropriate size as required to provide for the increase in toe and/or heel rigidity to lessen the downward motion of heel strike length and/or toe-off.

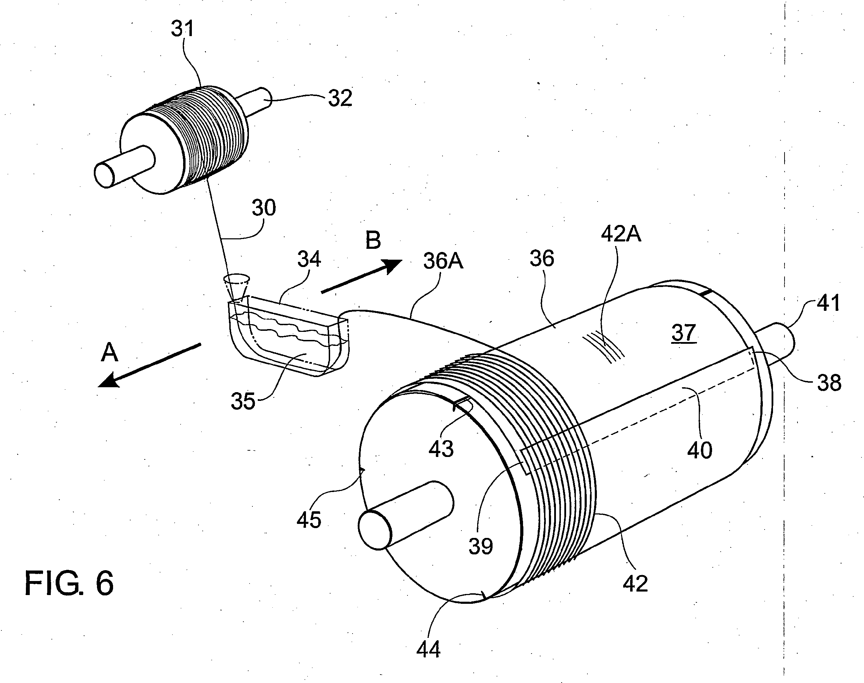

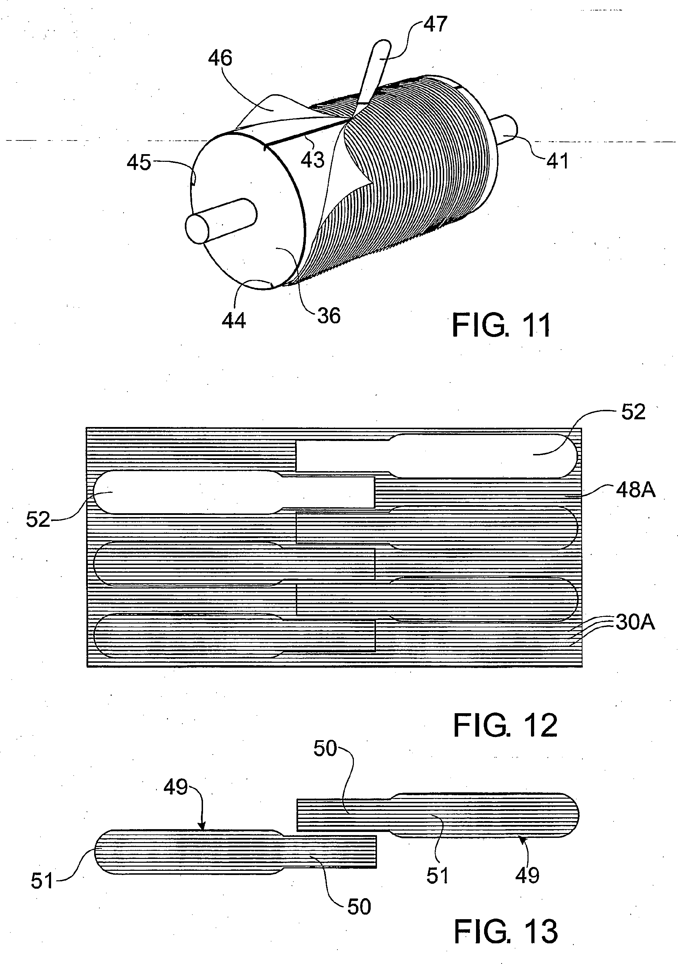

In relation to FIG. 6, it will be noted that carbon or glass filament 30 is unwound from a carbon bobbin 31 that is mounted and supported by axle 32. Axle 32 will rotate when the drum 36 pulls filament 30. Filament 30 is passed through a resin bath 34 containing the preferred epoxy resin solution 35. After passing through resin bath 34, the resin-impregnated filament 30A is then wound onto the drum 36 which has a sheet of release paper 37, preferably silicone coated release paper, attached thereto, wherein ends 38 and 39 may be overlapped and affixed together by double-sided tape at 40, so as to provide a continuous sheet of release paper 37 on drum 36. Resin-impregnated filament 30A may then be wound onto release paper 37 as drum 36 rotates. Drum 36 is being driven by suitable mechanical means (not shown) coupled to axle 41. Simultaneously as it rotates, the resin bath 34 together with axle 32 may travel in a linear direction indicated by arrows A or B causing the resin-impregnated filament 30A to be evenly spaced and laid (at a pre-determined fibre area weight or fibre density) onto release paper 37, creating an intermediate pre-preg sheet 48A (in FIG. 12) from successive loops 42, as shown. Each of the successive loops 42 will overlap the preceding loop as they are wound onto the release paper 37 by the rotation of drum 36. The result is an intermediate pre-preg sheet 48A (in FIG. 12) that has binding integrity of its fibres from each resin-impregnated filament 30A and the intermediate pre-preg sheet 48A can be separated from release paper 37. Overlapping resin-impregnated filaments 30A are shown constituting the final appearance of sheet 48A at 42A.

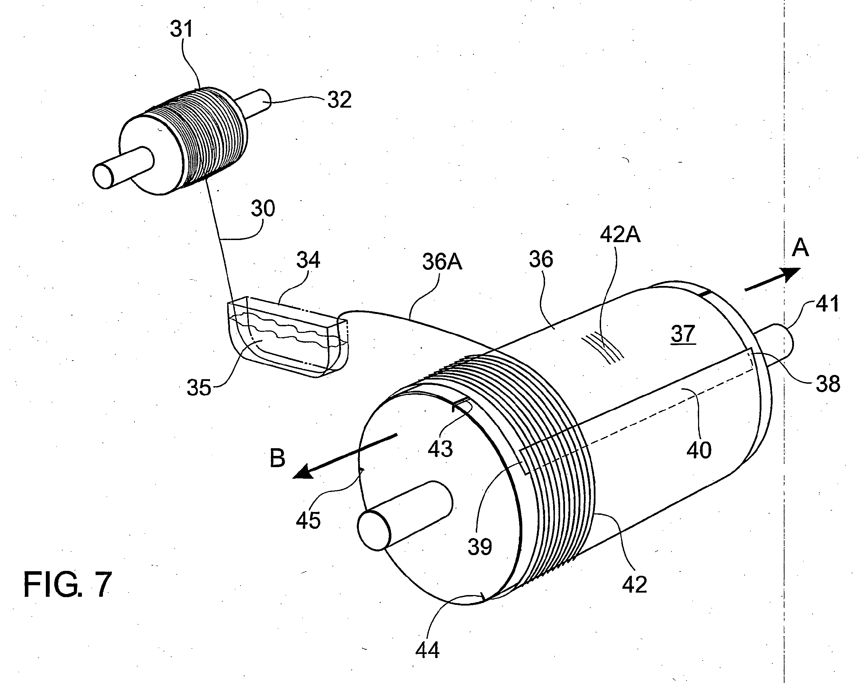

In an alternative arrangement, as shown in FIG. 7, the drum 36 may reciprocate in either direction A or B in relation to the stationary resin bath 34 and axle 32 so as to provide for the same result as described in FIG. 6. It will, however, be appreciated that the FIG. 6 arrangement is preferred.

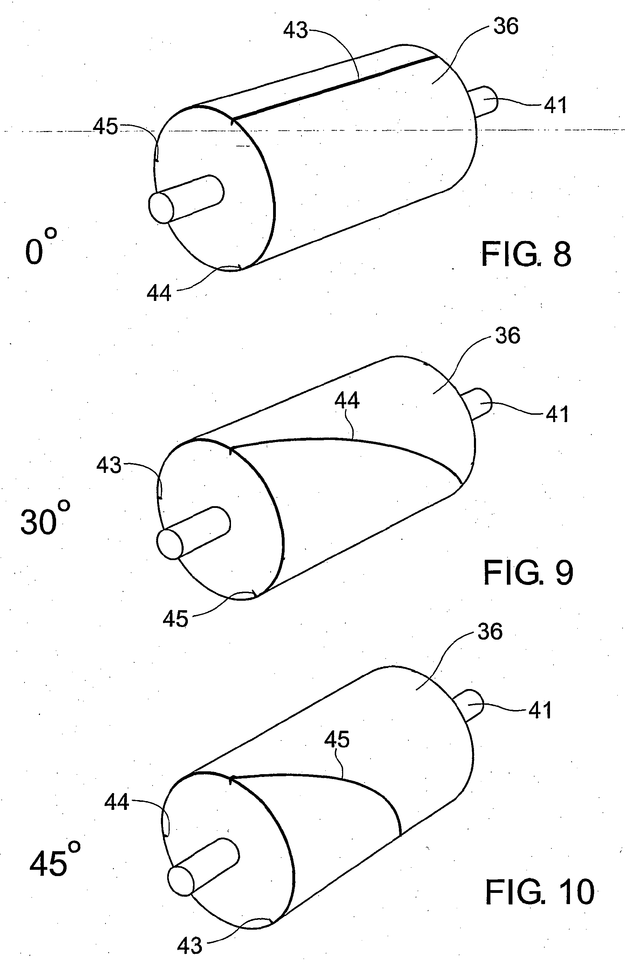

It will be noted from FIGS. 8, 9 and 10 that drum 36 may be provided with elongate grooves or cutting lines 43, 44 and 45 so as to facilitate cutting and removal of the composite sheet 46 from drum 36 at three cutting angles, typically but not exclusively at 0° or parallel to axle 41 for cutting line 43 as shown in FIG. 8, at 60° to axle 41 for cutting line 44 as shown in FIG. 9, or at 45° to axle 41 for cutting line 45 as shown in FIG.10. Cutting lines of other angles (although not shown in FIGS 8, 9 and 10) may be used to provide other intersecting angles for the unidirectional pre-pregs.

It will also be appreciated that cutting lines 43, 44, 45 or lines of other angles, may be provided on the same drum 36 or such cutting lines may be provided on different drums 36. However, having a plurality of cutting lines on the same drum is preferred for versatility and cost reasons.

FIGS. 11 to 13 show the cutting of composite sheet 46 by cutting knife 47 along cutting line 43 so as to provide for an intermediate pre-preg sheet 48A. The intermediate pre-preg sheet 48A is then cut to the preferred shape and sizes as shown in pre-preg 49. Each intermediate pre-preg sheet 48A can provide for multiple cuttings of pre-pregs 49 having longitudinal or 0° carbon or glass fibres as shown. The example shown in FIG. 13 illustrates pre-preg 49 having a narrow part 50 and a broader part 51 as required by the preferred design. The cutting and removal of pre-pregs 49 leaves gaps 52 in sheet 48A. Resin-impregnated filament 30A are shown in longitudinal orientation or 0°.

In a variation of the procedure described above, pre-pregs 49 may be formed not only by longitudinal orientation of the fibres (i.e.0°) but also by transverse or latitudinal orientation (i.e.90°) using intermediate pre-preg sheet 48A.

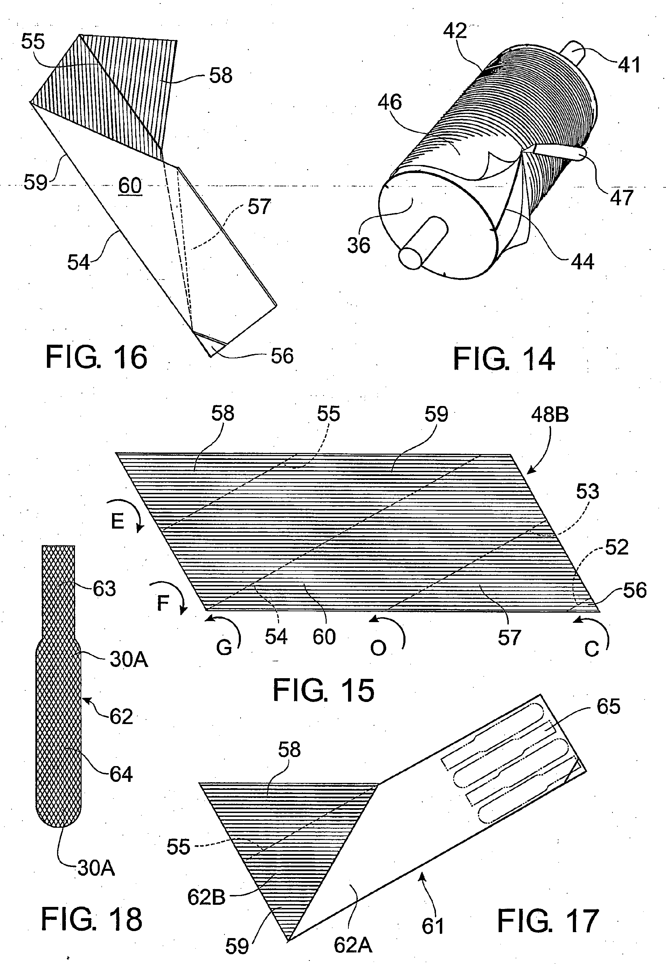

FIG. 14 shows the cutting of composite sheet 46 along cutting line 44 so as to provide for intermediate sheet 48B shown in FIG. 15, which is then provided with crease or fold lines 52, 53, 54 and 55. The folding begins with part 58 being folded along fold line 55 as shown by arrow E, followed by part 59 being folded along fold line 54, as shown by arrow F. Subsequently, part 57 is folded along fold line 53, as shown by arrow D, and lastly, part 56 is folded along fold line 52, as shown by arrow C.

Alternatively, part 60 may be folded along fold line 54, as indicated by arrow G, instead of part 59 along fold line 54 as indicated by arrow F. This procedure is further illustrated in FIGS. 16 and 17 to provide a final folded sheet 61 (shown in FIG. 17), from which multiple pre-pregs 62 (shown in FIG. 18) may be cut as shown in phantom in FIG. 17, leaving gaps 65. FIG. 18 also shows a pre-preg 62 having intersecting fibres from the resin-impregnated filaments 30A. The intersecting fibres are from the parts 56, 57, 60,59 and 58 being folded along fold lines 52, 53, 54 and 55 back upon themselves.

Optimum pliability rates, compressive strength, dynamic endurance, fore-and-aft and torsional rigidity in the various weight categories (30-130kg), may be achieved in the J-shaped pylon 11 and sole plate 23 (in FIG.1) by various carbon/carbon or carbon/glass fibre combinations with variations in fibre area weight and fibre orientation. All weight categories (30-130kg) in a preferred embodiment of the invention have the same number of layers of laminates in the construction.

The intrinsic characteristics and mechanical strength of the preferred carbon and glass fibres has created a variety of possible configurations in material compositions, fibre area weight and fibre orientations and sequencing to optimise the compressive strength, flexibility, durability, dynamic endurance, fore-and-aft and torsional rigidity in a preferred embodiment. Carbon fibre possesses much higher tensile strength and tensile modulus as compared to glass fibres. However, glass fibres, though less strong, have higher flexibility and fatigue endurance. Higher percentage of carbon fibres and lower percentage of glass fibres in the construction will result in higher stiffness and strength in a preferred embodiment and flexibility will increase with a reduction in tensile strength if we use a higher percentage of glass fibres.

Configurations of fibre orientation is another element in the present invention. Typically, placing longitudinal or 0° fibres along and parallel to the principle axis of J-shaped pylon 11 and sole plate 23 will result in very low torsional strength but very high stiffness, very high tensile and high compressive strength. Other fibre angles such, as 30°, 45° or 60° possess much higher torsional strength but lower tensile or compressive strength. It must be highlighted that the intrinsic mechanical properties of carbon and glass fibres are very different from each other and therefore it is natural to expect that their 0°, 30° and 45° angles produce very different results in torsion, tensile strength and compressive strength and this provides more options to configure and to optimise the compressive strength, flexibility, durability, dynamic endurance, fore-and-aft and torsion rigidity of each weight category in a preferred embodiment.

Typical carbon/carbon and carbon/glass compositions for the J-shaped pylon 11 and sole plate 23 are set out in the Table attached hereto.

The process of the invention ensures that all the fibres during resin impregnation are thoroughly "wetted" and properly impregnated with the epoxy resin. Typically, this method will produce carbon and glass pre-preg having a resin content range from 35% to 50%. The resin content is higher than most of the commercially available UD pre-preg. The epoxy resin functions as a glue to bind and encapsulate all fibres together. Higher resin content will ensure sufficient resin flow during moulding and will also provide a greater degree of control in the cross linking process, giving a much improved resin/fibre matrix. The result is an increase in strength and durability in a preferred embodiment.

All conventional lower limb prosthetic devices are constructed from UD longitudinal fibres in their main core with continuous and uninterrupted woven latitudinal or woven angular fibres on the outer layers to improve their torsion strength. The use of pre-preg 49 (having either 0° or 90° degree fiber orientation) and pre-preg 62 (having other intersecting fiber orientation) in the present preferred embodiment creates a totally new concept and method of construction for moulded products generally inclusive of the lower limb prosthetic device and is uniquely different from prior art methods.

In this present invention, fibre filaments do not have to be continuous and uninterrupted as shown by the method of producing pre-preg 49 and 62. It must be noted that the use of non-continuous fibre filaments constitutes a significant feature of the prosthetic device.

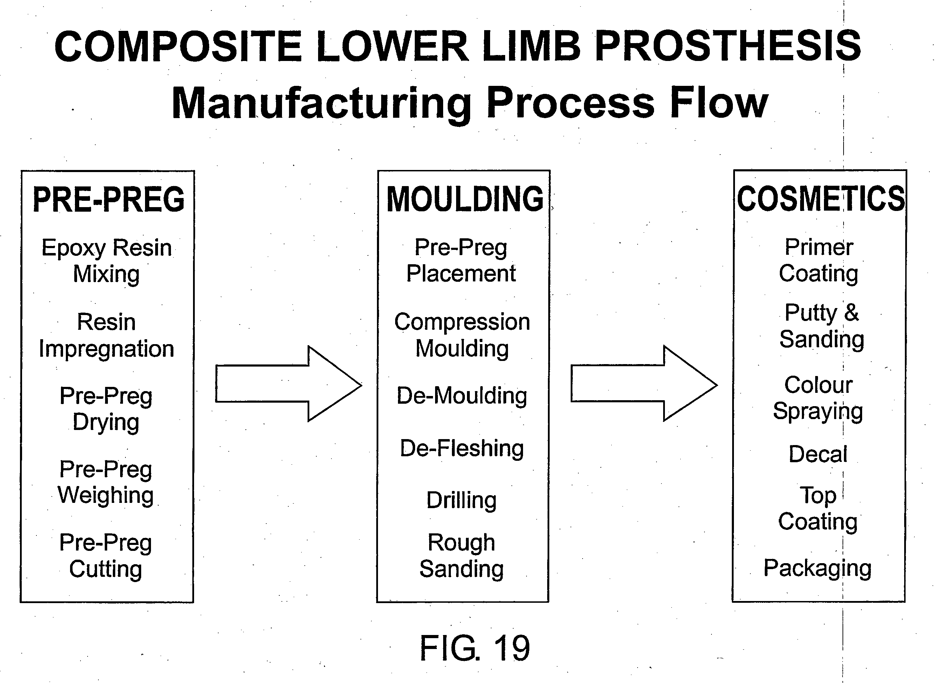

FIG. 19 refers to the preferred flow chart for the processes of the present invention in relation to manufacture of molded products inclusive of lower limb prosthetic device. Epoxy resin solution may be formed by mixing the resin, catalyst, additives and solvent in stipulated proportions. The epoxy resin solution may have a 60% to 70% solid content with a cure time of 30 min at 130° degree Celsius and may possess toughness, flexibility, good thermal stability and chemical resistance, and having good bonding strength with the carbon and glass fibres which are the preferred fibres for use in the present invention.

This method of the invention allows carbon and/or glass unidirectional pre-pregs to be produced with a wide selection of fibre area weights (ranging from 60 grams to 300 grams per square meter), resin content (ranging from 35% to 50% by weight) and fibre orientations and sequencing to be employ and it also provides the flexibility to "choose and optimize" pre-pregs from the various combinations of material compositions, fibre area weight, fibre orientation and resin content that are available.

Typically, the carbon and glass fibres used in the preferred method of the present invention, are high strength filaments. The preferred drum wound technique ensures that all the fibres during resin impregnation are thoroughly "wetted" and properly impregnated with the epoxy resin. With adroit control in resin viscosity during the resin impregnation process, it is possible to control resin content within +2%. Controlled and consistent resin content are preferable in the maintenance of compressive strength, durability, dynamic endurance, pliability rates, fore-and-aft and torsional rigidity.

The intermediate pre-preg sheets 48A and final folded pre-preg sheet 61 may be subjected to force drying in an oven to remove solvents and any other unwanted volatile components in the epoxy resin. Forced drying also makes the pre-preg sheets less tacky and more suitable for handling in the lay-up process. Suitable oven specifications are temperature of 50-65°C and drying time of 3 to 4 hours. Subsequently after the forced drying process, each intermediate pre-preg sheet 48A and final folded pre-preg sheet 61 may be weighed and cut into pre-preg 49 or pre-preg 62 as appropriate.

It must be highlighted that weighing of the intermediate pre-preg sheets 48A and final folded pre-preg sheet 61 after forced drying is a desirable aspect of the process of the invention. This is to segregate pre-pregs that falls in the high or low end of the resin content range, and to combine these pre-pregs appropriately so that the weight of the moulded J-shaped pylon 11 or sole plate 23 remains relatively constant. As an example, a pre-preg having high resin content may be used in conjunction with another that has low resin content during lay-up process.

After the lay-up of successive layers of pre-preg 49 and pre-preg 62 in the respective compression mould cavity, the compression mould may be heated to 135°C-150°C (and more preferably 140°C) suitably in combination with a hydraulic pressure of 200 kg/m² using a 200mm diameter hydraulic piston.

After curing, de-moulding is undertaken to remove the moulded members, which are then prepared for assembly and final cosmetic treatment.

It will be appreciated from the foregoing that in view of the fact that the moulded product can be made from a mould cavity of similar dimensions regardless of weight that this feature will also apply in a preferred embodiment to the moulded product such as the J shaped pylon or the sole plate.

It therefore will be appreciated from the foregoing that the process of the invention provides a lower leg prosthesis which has considerable advantages over the prior art as will be apparent from the foregoing description.

### EXAMPLES

### EXAMPLE I

A preferred method of lay up or sequencing of both pre-pregs 49 and 62 is described below in relation to manufacture of a J shaped pylon.

All weight categories (30kg-130kg), in this preferred method, have the same number (58) of layers with variations to the fibre sequencing and fiber orientation to achieve the varied pliability rates for each weight category, viz:
30kg to 40kg - pylon 345g ±5g, 38 layers of 60° fiber and 20 layers of 0° and 90° fiber combination.
40kg to 50kg - pylon 345g ±5g, 38 layers of 60° fiber and 20 layers of 0° fiber combination.
50kg to 60kg - pylon 345g ±5g, 38 layers of 45° and 60° fiber combination with 20 layers of 0° and 90° fiber combination.
60kg to 70kg - pylon 345g ±5g, 38 layers of 45° and 60° fiber combination and 20 layers of 0° fiber.
70kg to 80kg - pylon 345g ±5g, 38 layers of 45° and 20 layers of 0° and 90° fiber combination.
80kg to 90kg - pylon 345g ±5g, 38 layers of 45° and 20 layers of 0° fiber.
90kg to 100kg - pylon 345g ±5g, 38 layers of 30° and 45° fiber combination and 20 layers of 0° and 90° fiber combination.
100kg to 110kg - pylon 345g ±5g, 38 layers of 30° and 45° fiber combination and 20 layers of 0° fiber.
110kg to 120kg - pylon 345g ±5g, 38 layers of 30° fiber and 20 layers of 0° and 90° fiber combination 120kg to 130kg - pylon 345g ±5g, 38 layers of 30° fiber and 20 layers of 0° fiber.

For all pylon categories (30-130kg), the carbon/carbon pre-preg lay-up sequences are preferably the same. Thus, for the pylon having 58 layers which comprise 38 layers of angled fiber and 20 layers of 0° and 90° fiber combination, the lay-up sequence is 8 layers of angled carbon fiber with FAW 100 at 0.12mm, 6 layers of carbon 0° and 90° combination with FAW 150 at 0.15mm, 10 layers of angled carbon fiber with FAW (Fibre Area Weight) 100 at 0.12mm, 6 layers of carbon 0° and 90° combination with FAW 150 at 0.15mm, 10 layers of angled carbon fiber with FAW 100 at 0.12mm, 8 layers of carbon 0° and 90° combination with FAW 150 at 0.15mm and 10 layers of angled carbon fiber with FAW 100 at 0.12mm.

### EXAMPLE II

Maximisation of pliability rates, dynamic endurance, compressive strength, fore-and-aft and torsional rigidity of the different weight categories (30-130kg) may be achieved in the sole plate by a combination of carbon fibre angles (0°, 30°, 45°, 60° and 90°) and glass fibre angles (30°, 45° and 60°), varied fibre lengths, fibre alignment (using lateral, transverse and diagonal directions), combined carbon layer thicknesses (0.12mm FAW 100/sq.m and 0.15mm FAW 150g/sq.m) and/or glass fibre layer thickness (0.16mm FAW 126g/sq.m).

All weight categories discussed above (30kg to 130kg) preferably have the same total number (52) of layers for either the entire carbon or combined carbon/glass fibre sole plate. Through the variations in the lay-up sequencing and fibre alignment for both carbon/carbon and carbon/glass fibre, the varied pliability rates for each weight category have been achieved, viz:
30kg to 40kg - Sole Plate 190g ±5g, Carbon/Glass 50%/50%
40kg to 50kg - Sole Plate 190g ±5g, Carbon/Glass 50%/50%
50kg to 60kg - Sole Plate 190g ±5g, Carbon/Glass 70%/30%
60kg to 70kg - Sole Plate 190g ±5g, Carbon/Glass 70%/30%
70kg to 80kg - Sole Plate 190g ±5g, Carbon 100%
80kg to 90kg - Sole Plate 190g ±5g, Carbon 100%
90kg to 100kg - Sole Plate 190g ±5g, Carbon 100%
100kg to 110kg - Sole Plate 190g ±5g, Carbon 100%
110kg to 120kg - Sole Plate 190g ±5g, Carbon 100%
120kg to 130kg - Sole Plate 190g ±5g, Carbon 100%

For the following sole plate weight categories (30-130kg), the carbon/carbon and carbon/glass pre-preg lay-up sequence having regard to pre pegs 49 and 62 are:
30 to 40kg - 16 layers of 60° carbon fiber with FAW 100 at 0.12mm, 10 layers of carbon 0° and 90° combination with FAW 150 at 0.15mm and 26 layers of 60° glass fiber with FAW 126 at 0.16mm
40 to 50kg - 16 layers of 60° carbon fiber with FAW 100 at 0.12mm, 10 layers of carbon 0° with FAW 150 at 0.15mm and 26 layers of 60° glass fiber with FAW 126 at 0.16mm
50 to 60kg -26 layers of 60° carbon fiber with FAW 100 at 0.12mm, 10 layers of carbon 0° and 90° combination with FAW 150 at 0.15mm and 16 layers of 60° glass fiber with FAW 126 at 0.16mm
60 to 70kg - 26 layers of 60° carbon fiber with FAW 100 at 0.12mm, 10 layers of carbon 0° with FAW 150 at 0.15mm and 16 layers of 60° glass fiber with FAW 126 at 0.16mm
70 to 80kg - 36 layers of 45° and 60° carbon fiber combination with FAW 100 at 0.12mm, 16 layers of carbon 0° and 90° combination with FAW 150 at 0.15mm
80 to 90kg - 36 layers of 45° and 60° carbon fiber combination with FAW 100 at 0.12mm, 16 layers of carbon 0° with FAW 150 at 0.15mm
90 to 100kg - 36 layers of 45° carbon fiber with FAW 100 at 0.12mm, 16 layers of carbon fiber 0° and 90° carbon fiber combination with FAW 150 at 0.15mm
100 to 110kg - 36 layers of 45° carbon fiber with FAW 100 at 0.12mm, 16 layers of carbon fiber 0° with FAW 150 at 0.15mm
110 to 120 kg - 36 layers of 30° and 45° carbon fiber combination with FAW 100 at 0.12mm, 16 layers of carbon fiber 0° with FAW 150 at 0.15mm
120 to 130 kg - 36 layers of 30° carbon fiber with FAW 100 at 0.12mm, 16 layers of carbon fiber 0° with FAW 150 at 0.15mm.

**TABLE**

| WEIGHT CLASS (kg) | J-SHAPED PYLON (11) | SOLE PLATE (23) |
|---|---|---|
| 30-40 | 100% carbon | 50% carbon/50% glass |
| 40-50 | 100% carbon | 50% carbon/50% glass |
| 50-60 | 100% carbon | 70% carbon/30% glass |
| 60-70 | 100% carbon | 70% carbon/30% glass |
| 70-80 | 100% carbon | 100% carbon |
| 80-90 | 100% carbon | 100% carbon |
| 90-100 | 100% carbon | 100% carbon |
| 100-110 | 100% carbon | 100% carbon |
| 110-120 | 100% carbon | 100% carbon |
| 120-130 | 100% carbon | 100% carbon |

## Claims

1. A method of construction of a moulded product which includes the steps of:
(a) impregnating non-metallic fibres (30) in epoxy resin (34) whereby said impregnated fibres (30A) may be arranged to form a composite sheet (46) of said fibres; and
(b) cutting a plurality of pre-pregs (49) having fibres arranged in a longitudinal, i.e. 0 degrees, or latitudinal orientation, i.e. 90 degrees, from an intermediate sheet (48A) obtained by cutting said composite sheet (46) formed in step (a) at an angle of 0 degrees; or
(b') cutting the composite sheet (46) formed in step (a) at a variety of different angles selected from the group consisting of 15 degrees, 22 ½ degrees, 30 degrees, 45 degrees and 60 degrees so to obtain an intermediate sheet (48B) having sloping sides with said angle to the vertical; folding the intermediate sheet upon itself to form a final folded sheet (61); cutting a plurality of pre-pregs (62) having fibres arranged in intersecting orientation from said final folded sheet (61); and
(c) forming successive layers of pre-pregs (49, 62) alternating pre-pregs (49) obtained from step (b) and pre-pregs (62) obtained from step (b') so that said layers are arranged in stacked relationship in a mould cavity of a compression mould; and
(d) compression moulding at elevated temperature; and
(e) removing the moulded product from the compression mould;
wherein in step (c), pre-pregs with various combinations of carbon/carbon or carbon/glass fibres with variations in fibre area weight and fibre orientation are used so that step (d) is carried out in a mould cavity having the same dimensions for different weights or different weight categories of moulded product.

2. A method as claimed in claim 1 wherein in step (a) use is made of a drum winding technique whereby said impregnated fibres (30A) are wound onto a drum (36) so as to provide the composite sheet (46) formed of the fibres (30A) supported on a sheet (37) of release paper on the drum (36), whereby upon removal of the release paper (37), the intermediate sheet (48A, 48B) used in steps (b) or (b') is obtained by cutting the composite sheet (46) as it is supported on the drum (36).

3. A method as claimed in claim 2 wherein the cutting of the composite sheet (46) is carried out by provision of cutting lines or grooves (43, 44, 45) which are formed in an outer surface of the drum (36) at an angle selected from the group consisting of 15 degrees, 22 ½ degrees, 30 degrees, 45 degrees and 60 degrees for obtaining the pre-pregs (62) of intersecting fibres in step (b') and 0 degrees for obtaining the pre-pregs (49) of longitudinal and latitudinal fibres in step (b).

4. A method as claimed in claim 1 wherein use is made of a hot melt technique wherein after hot melt resin impregnation of the fibres they are laid onto a continuous sheet of release paper to obtain the composite sheet (46) and subsequently stored as rolls.

5. A method as claimed in claim 1 wherein in step (a) the composite sheet (46) has all of its fibres (30A) arranged in the same or similar orientation or direction.

6. A method as claimed in claim 1 wherein in step (b) each pre-preg (49) has all of its fibres (30A) arranged in a longitudinal orientation.

7. A method as claimed in claim 1 wherein in step (b') each pre-preg (62) has all of its fibres (30A) arranged in an intersecting orientation.

8. A method as claimed in claim 1 wherein in step (b') the angle at which the composite sheet (46) is cut is selected from 30 degrees and 45 degrees.

9. A method as claimed in claim 1 wherein in step (a) the composite sheet (46) is formed by longitudinally and/or latitudinally oriented fibres (30A) so as to provide a sheet which is in the shape of a rectangle or square.

## Patentansprüche

1. Verfahren zum Aufbau eines Formprodukts, umfassend die folgenden Schritte:
(a) Imprägnieren von nichtmetallischen Fasern (30) in Epoxidharz (34), wobei die imprägnierten Fasern (30A) so angeordnet werden können, dass sie einen Verbundbogen (46) der Fasern bilden; und
(b) Schneiden von mehreren Prepregs (49) mit Fasern, die in einer Längs-, d.h., 0 Grad, oder in einer Querausrichtung, d.h., 90 Grad, angeordnet sind, aus einem Zwischenbogen (48A), der durch Schneiden des in Schritt (a) gebildeten Verbundbogens (46) in einem Winkel von 0 Grad erhalten wurde; oder
(b') Schneiden des in Schritt (a) gebildeten Verbundbogens (46) in einer Vielzahl von Winkeln, die aus der Gruppe gewählt ist, welche aus 15 Grad, 22 ½ Grad, 30 Grad, 45 Grad und 60 Grad besteht, um einen Zwischenbogen (48B) mit schrägen Seiten mit dem genannten Winkel zu der Senkrechten zu erhalten; Falten des Zwischenbogens auf sich selbst, um einen endgültigen gefalteten Bogen (61) zu erhalten; Schneiden von mehreren Prepregs (62) mit Fasern, die in einer sich überkreuzenden Ausrichtung angeordnet sind, aus dem endgültigen gefalteten Bogen (61); und
(c) derartiges Bilden von aufeinanderfolgenden Schichten von Prepregs (49, 62), wobei Prepregs (49), die durch Schritt (b) erhalten wurden, und Prepregs (62), die durch Schritt (b') erhalten wurden, abgewechselt werden, dass die Schichten in einer gestapelten Beziehung in einem Formhohlraum einer Pressform angeordnet werden; und
(d) Pressformen bei einer erhöhten Temperatur; und
(e) Entnehmen des Formprodukts aus der Pressform;
wobei in Schritt (c) Prepregs mit verschiedenen Kombinationen von Kohlenstoff-/Kohlenstofffasern oder Kohlenstoff-/Glasfasern mit Unterschieden hinsichtlich des Faserflächengewichts und der Faserausrichtung verwendet werden, so dass der Schritt (d) in einem Formhohlraum ausgeführt wird, der für unterschiedliche Gewichte oder unterschiedliche Gewichtskategorien des Formprodukts die gleichen Abmessungen aufweist.

2. Verfahren nach Anspruch 1, wobei in Schritt (a) von einer Trommelwicklungstechnik Gebrauch gemacht wird, wobei die imprägnierten Fasern (30A) so auf eine Trommel (36) gewickelt werden, dass der Verbundbogen (46), der aus den auf einem Bogen (37) aus Trennpapier auf der Trommel (36) getragenen Fasern (30A) gebildet ist, erbracht wird, wobei nach Entfernen des Trennpapiers (37) der Zwischenbogen (48A, 48B), der in Schritt (b) oder (b') verwendet wird, erhalten wird, indem der Verbundbogen (46) geschnitten wird, während er auf der Trommel (36) gehalten wird.

3. Verfahren nach Anspruch 2, wobei das Schneiden des Verbundbogens (46) durchgeführt wird, indem Schnittlinien oder Nuten (43, 44, 45) vorgesehen sind, die in einer Außenfläche der Trommel (36) in einem Winkel gebildet sind, der zum Erhalt der Prepregs (62) in Schritt (b') aus sich überkreuzenden Fasern aus der Gruppe gewählt ist, die aus 15 Grad, 22 ½ Grad, 30 Grad, 45 Grad und 60 Grad besteht, und zum Erhalt der Prepregs (49) aus Längs- und Querfasern in Schritt (b) 0 Grad beträgt.

4. Verfahren nach Anspruch 1, wobei von einer Heißschmelztechnik Gebrauch gemacht wird, wobei die Fasern nach ihrer Imprägnierung mit heißem geschmolzenem Harz auf einen fortlaufenden Bogen aus Trennpapier gelegt werden, um den Verbundbogen (46) zu erhalten, und anschließend als Rollen aufbewahrt werden.

5. Verfahren nach Anspruch 1, wobei in Schritt (a) alle Fasern (30A) des Verbundbogens (46) in der gleichen oder einer ähnlichen Ausrichtung oder Richtung angeordnet sind.

6. Verfahren nach Anspruch 1, wobei in Schritt (b) alle Fasern (30A) jedes Prepregs (49) in einer Längsausrichtung angeordnet sind.

7. Verfahren nach Anspruch 1, wobei in Schritt (b') alle Fasern (30A) jedes Prepregs (62) in einer sich kreuzenden Ausrichtung angeordnet sind.

8. Verfahren nach Anspruch 1, wobei in Schritt (b') der Winkel, in dem der Verbundbogen (46) geschnitten wird, aus 30 Grad und 45 Grad gewählt wird.

9. Verfahren nach Anspruch 1, wobei in Schritt (a) der Verbundbogen (46) so durch längs und/oder quer ausgerichtete Fasern (30A) gebildet wird, dass ein Bogen erbracht wird, der die Form eines Rechtecks oder eines Quadrats aufweist.

## Revendications

1. Procédé de construction d'un produit moulé qui comprend les étapes consistant à :
(a) imprégner des fibres non métalliques (30) dans une résine époxyde (34) moyennant quoi lesdites fibres imprégnées (30A) peuvent être disposées pour former une feuille composite (46) desdites fibres ; et
(b) découper une pluralité de préimprégnés (49) ayant des fibres disposées dans une orientation longitudinale, c'est-à-dire 0 degré, ou latitudinale, c'est-à-dire 90 degrés, par rapport à une feuille intermédiaire (48A) obtenue en coupant ladite feuille composite (46) formée dans l'étape (a) selon un angle de 0 degré ; ou
(b') découper la feuille composite (46) formée dans l'étape (a) selon une diversité d'angles différents choisis dans le groupe constitué de 15 degrés, 22½ degrés, 30 degrés, 45 degrés et 60 degrés de façon à obtenir une feuille intermédiaire (48B) ayant des côtés inclinés avec ledit angle par rapport à la verticale ; plier la feuille intermédiaire sur elle-même pour former une feuille pliée finale (61) ; découper une pluralité de préimprégnés (62) ayant des fibres disposées dans une orientation qui se croise par rapport à ladite feuille pliée finale (61) ; et
(c) former des couches successives de préimprégnés (49,62) en alternant les préimprégnés (49) obtenus par l'étape (b) et les préimprégnés (62) obtenus par l'étape (b') de sorte que lesdites couches sont disposées dans une relation empilée dans une cavité de moule d'un moule de compression ; et
(d) mouler par compression à température élevée ; et
(e) retirer le produit moulé du moule de compression ;
dans lequel, dans l'étape (c), on utilise des préimprégnés avec diverses combinaisons de fibre de carbone/carbone ou de carbone/verre avec des variations de masse surfacique de fibre et d'orientation de fibre de sorte que l'étape (d) est effectuée dans une cavité de moule ayant les mêmes dimensions pour différents poids ou différentes catégories de poids de produit moulé.

2. Procédé selon la revendication 1, dans lequel, dans l'étape (a), on a recours à une technique d'enroulement sur tambour selon lequel lesdites fibres imprégnées (30A) sont enroulées sur un tambour (36) de façon à fournir la feuille composite (46) formée des fibres (30A) supportées sur une feuille (37) de papier détachable sur le tambour (36), selon quoi lors du retrait du papier détachable (37), la feuille intermédiaire (48A, 48B) utilisée dans les étapes (b) ou (b') est obtenue en coupant la feuille composite (46) lorsqu'elle est soutenue sur le tambour (36).

3. Procédé selon la revendication 2, dans lequel la découpe de la feuille composite (46) est effectuée en réalisant des lignes de coupe ou des rainures (43, 44, 45) qui sont formées dans une surface externe du tambour (36) selon un angle choisi dans le groupe constitué de 15 degrés, 22½ degrés, 30 degrés, 45 degrés et 60 degrés pour obtenir les préimprégnés (62) de fibres qui se croisent dans l'étape (b') et 0 degré pour obtenir les préimprégnés (49) de fibres longitudinales et latitudinales dans l'étape (b).

4. Procédé selon la revendication 1, dans lequel on a recours à une technique par thermofusible dans laquelle après imprégnation de résine thermofusible des fibres, elles sont déposées sur une feuille continue de papier détachable pour obtenir la feuille composite (46) et sont par la suite stockées sous forme de rouleaux.

5. Procédé selon la revendication 1, dans lequel, dans l'étape (a), la feuille composite (46) a toutes ses fibres (30A) disposées dans la même orientation ou direction ou une orientation ou direction similaire.

6. Procédé selon la revendication 1, dans lequel, dans l'étape (b), chaque préimprégné (49) a toutes ses fibres (30A) disposées dans une orientation longitudinale.

7. Procédé selon la revendication 1, dans lequel, dans l'étape (b'), chaque préimprégné (62) a toutes ses fibres (30A) disposées dans une orientation qui se croise.

8. Procédé selon la revendication 1, dans lequel, dans l'étape (b'), l'angle selon lequel est coupée la feuille composite (46) est choisi parmi 30 degrés et 45 degrés.

9. Procédé selon la revendication 1, dans lequel, dans l'étape (a), la feuille composite (46) est formée par des fibres orientées de façon longitudinale et/ou latitudinale (30A) de façon à fournir une feuille qui est sous la forme d'un rectangle ou d'un carré.
